# EUROPEAN PATENT SPECIFICATION

(11) **EP 2 937 108 B1**
(45) Date of publication and mention of the grant of the patent: **13.03.2019**
(21) Application number: 15161844.4
(22) Date of filing: 31.03.2015
(51) Int. Cl.: A61M 25/01, A61M 25/10, A61M 25/00

(54) **CATHETER**
KATHETER
CATHÉTER

(30) Priority: 25.04.2014 JP 2014090784
(43) Date of publication of application: 28.10.2015
(73) Proprietor: Asahi Intecc Co., Ltd., Seto-shi, Aichi 489-0071 (JP)
(72) Inventor: Yagi, Takayuki, Seto-shi, Aichi 489-0071 (JP)
(74) Representative: Winter, Brandl, Fürniss, Hübner, Röss, Kaiser, Polte - Partnerschaft mbB

(56) References cited:
- JP-A- 2001 190 680
- JP-A- 2005 230 318

## Description

### 1. Field of the Invention

The present invention relates to a catheter capable of passing a hard lesion, for example.

### 2. Description of the Related Art

There is conventionally proposed a catheter including a metallic distal end tip (Japanese Unexamined Patent Application No. 2007-244492, for example). In such a catheter, the distal end portion has sufficient hardness. Thus, the catheter is used preferably as a catheter inserted into a hard lesion formed inside a blood vessel, a bile duct, and a pancreatic duct, for example, or a catheter inserted into a bone to suck bone marrow.

Moreover, there is proposed a catheter in which a resin distal end tip is provided with a groove so as to have flexibility (U.S. Unexamined Patent Application No. 2008/0255507, for example).

A slit is provided on a metallic distal end tip using the above-described conventional technique, whereby the distal end tip can be bent easily while the hardness thereof is maintained. This realizes a catheter being excellent in passing performance relative to a hard lesion, for example, and having favorable following properties for a tortuous blood vessel such as a peripheral blood vessel. Further, document JP2001190680 (A) introduces a medical tube comprising a cylindrical layer (resin layer) having a first resin part and five strip-like second resin parts. Document JP2005230318 (A) introduces a catheter a catheter formed by a tube, a metal mesh would around the tube, and a coating covering around the metal mesh.

However, when a slit is provided on the metallic distal end tip, unevenness occurs on the surface of the distal end tip, which causes a problem that the distal end tip is easily caught on a lesion, for example.

### SUMMARY OF THE INVENTION

The invention is defined as in the appended claims. In view of the above problem of the conventional technique, the present invention aims at allowing, in a catheter including a metallic distal end tip, the distal end tip to bend easily, and making it difficult for the distal end tip to be caught on a lesion, for example.

In order to solve the problem described above, the catheter of the invention adopts the following structure. That is, the catheter includes a tube body constituted by an inner layer, a reinforcing body covering the inner layer, and an outer layer covering the reinforcing body, and a tubular metallic distal end tip provided at a distal end of the tube body, in which a slit is formed on the distal end tip and penetrates the distal end tip (50) from an outer peripheral surface of the distal end tip (50) to an inner surface of the distal end tip (50), and an outer peripheral surface of the distal end tip is provided with outer coating, and the outer coating enters the inside of the slit.

In such a catheter of the invention, the metallic distal end tip is provided with the slit, thus allowing the distal end tip to bend easily. Moreover, an outer peripheral surface of the distal end tip is covered with outer coating, which smoothens the surface of the distal end tip and then makes it difficult for the distal end tip to be caught on a lesion, for example.

In particular, the outer coating enters the inside of the slit to be firmly fixed to the distal end tip (an anchoring effect), which makes it possible to securely maintain the smooth surface of the distal end tip.

The slit formed on the distal end tip may be formed helically around a longitudinal axis of the distal end tip. Further, a pitch of the slit may decrease toward a distal end of the distal end tip.

Moreover, in the catheter of the invention, inner coating may be provided on an inner peripheral surface of the distal end tip, so that the outer coating entering the inside of the slit comes into contact with the inner coating.

In such a catheter of the invention, the distal end tip is covered with outer coating and inner coating, and the outer coating entering the inside of the slit and the inner coating are in close contact with each other. Thus, the outer coating (and the inner coating) can be fixed to the distal end tip more firmly.

Moreover, the inner coating can also smoothen the inner peripheral surface of the distal end tip, which also prevents the case in which a medical instrument (a guide wire, for example) passing a lumen of the catheter is caught on the inner peripheral surface of the distal end tip.

Moreover, in the catheter of the invention described above, the outer coating and the outer layer of the tube body may be formed integrally, and the inner coating and the inner layer of the tube body may be formed integrally.

In such a catheter of the invention, the outer coating and the outer layer of the tube layer are formed integrally, and the inner coating and the inner layer of the tube body are formed integrally, whereby a connection portion between the distal end tip and the tube body is coated seamlessly. This prevents occurrence of catch with the outside (a lesion, for example) and the inside (a guide wire passing a lumen of the catheter, for example) at the connection portion between the distal end tip and the tube body. At the same time, it is also possible to enhance connection strength between the distal end tip and the tube body.

Moreover, in the catheter of the invention, a braid layer may be used as a reinforcing body.

Alternatively, a coil body formed by winding at least one metallic wire into a helical coil structure may be used as a reinforcing body, and the distal end tip may be welded to the distal end of the coil body.

In such a catheter of the invention, the coil body and the distal end tip are joined by welding. Thus, it is possible to further enhance connection strength between the distal end tip and the tube body.

Further, in such a catheter the coil body may be formed by winding a single metallic wire or by winding a plurality of metallic wires.

Furthermore, in the catheter of the invention, the distal end of the coil body as a reinforcing body may include a part formed integrally by mutually-melted wires constituting the coil body, and the metallic distal end tip may be welded to the part.

In such a catheter of the invention, the wires are mutually melted and formed integrally at the distal end portion of the coil body, which regulates the movement of the wires at the distal end portion of the coil body. As a result, it is possible to easily weld the distal end tip and the distal end portion of the coil body.

### BRIEF DESCRIPTION OF THE DRAWINGS

FIG. 1 is an explanatory diagram illustrating a structure of a catheter according to a first embodiment of the invention;
FIG. 2 is an enlarged view illustrating the vicinity of a distal end of the catheter (an area A in FIG. 1) according to the first embodiment of the invention, in which (a) illustrates a cross section illustrating the vicinity of the distal end of the catheter, and (b) illustrates an outer view of a distal end tip;
FIG. 3 is an enlarged view illustrating the vicinity of a distal end of a catheter according to a second embodiment of the invention;
FIG. 4 is an enlarged view illustrating the vicinity of a distal end of a catheter according to a third embodiment of the invention;
FIG. 5 is an enlarged view illustrating the vicinity of a distal end of a catheter according to a fourth embodiment of the invention; and
FIG. 6 is an enlarged view illustrating the vicinity of a distal end of a catheter according to a fifth embodiment of the invention.

### DETAILED DESCRIPTION OF THE PREFERRED EMBODIMENTS

### A: First embodiment:

In the following, various embodiments of the catheter of the invention will be described in order to clarify the subject matter of the above-described invention.

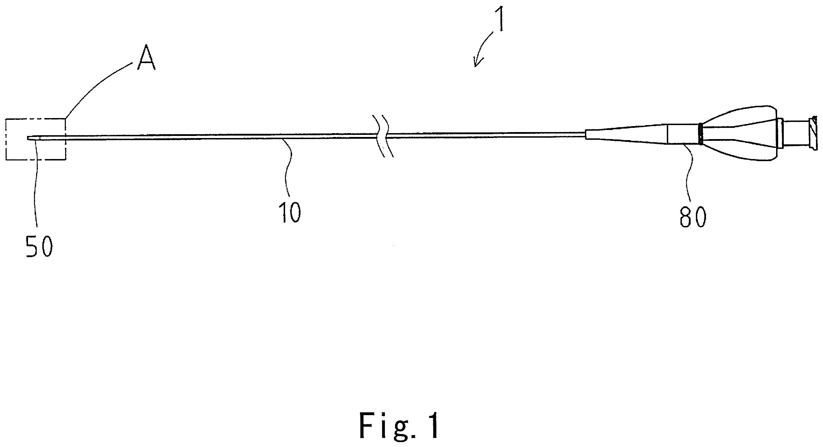

FIG. 1 is an explanatory diagram illustrating a structure of a catheter 1 according to a first embodiment of the invention. As illustrated in FIG. 1, the catheter 1 of the first embodiment is constituted by a catheter shaft 10, a connector 80 provided at the proximal end portion of the catheter shaft 10, and a distal end tip 50 provided at the distal end portion of the catheter shaft 10.

Note that the "catheter shaft" in the specification corresponds to the "tube body" of the invention.

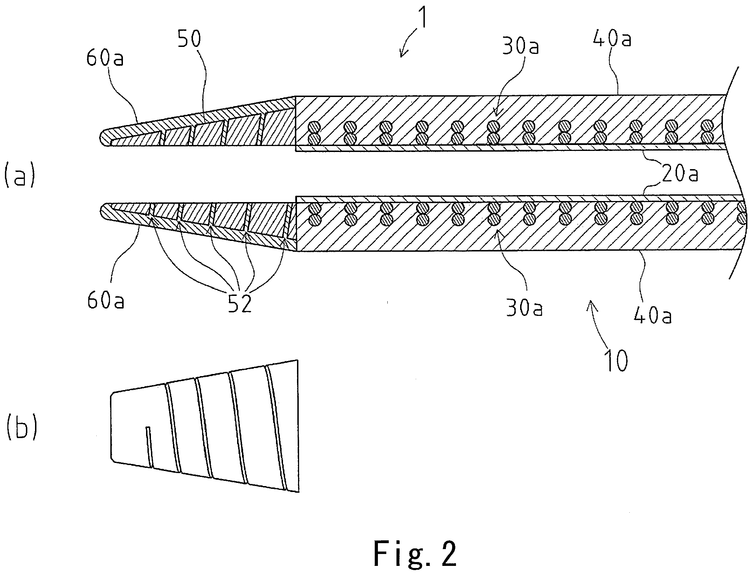

FIG. 2 is an enlarged view illustrating the vicinity of a distal end of the catheter 1 (an area A in FIG. 1) according to the first embodiment of the invention. As illustrated in FIG. 2(a), in the catheter 1 of the first embodiment, the catheter shaft 10 is a tubular structural body, and is constituted by an inner layer 20a, a braid layer 30a covering the inner layer 20a, and an outer layer 40a covering the braid layer 30a. Note that the "braid layer" in the specification is one form of the "reinforcing body" of the invention.

The inner layer 20a is formed of resin. The resin material forming the inner layer 20a is not especially limited. However, considering slidability with an inserted instrument (a guide wire or a catheter, for example), polytetrafluoroethylene (PTFE) is preferable.

The braid layer 30a is formed by braiding single wires. The material of the wires forming the braid layer 30a is not especially limited, and there can be used stainless steel (SUS304, SUS316, etc.), gold, white gold, tungsten, platinum, nickel, and an alloy of such elements, for example.

The outer layer 40a is formed of resin. The resin material forming the outer layer 40a is not especially limited, and there can be used polyamide, polyamide elastomer, polyester, and polyurethane, for example.

The metallic distal end tip 50 is provided at the distal end portion of the catheter shaft 10. The distal end tip 50 of the first embodiment is formed to be tapered in outer diameter toward the distal end. This improves passing performance relative to a hard lesion (a calcified lesion, for example).

Note that the metallic material used for the distal end tip 50 is not especially limited, and there can be used stainless steel (SUS304, SUS316, etc.), gold, white gold, tungsten, platinum, nickel, and an alloy of such elements, for example. However, the distal end tip 50 is preferably formed of radiopaque metallic material so that a position of the distal end of the catheter 1 can be grasped in a radioscopic image.

Moreover, the distal end tip 50 of the first embodiment is provided with a slit 52. As it is seen from Fig. 2 the slit 52 penetrates the distal end tip 50 from an outer peripheral surface of the distal end tip 50 to an inner surface of the distal end tip 50. The slit 52 is provided to lighten the metallic distal end tip 50 and thus improve flexibility thereof.

Note that as illustrated in FIG. 2(b), the slit 52 is formed helically on the distal end tip 50 in the catheter 1 of the first embodiment. Thus, the distal end tip 50 can be bent in any direction with a same level of easiness. Moreover, the pitch of the slit 52 is formed to be smaller toward the distal end, and the distal end area of the distal end tip 50 is especially flexible. In this manner, it is possible to reduce the risk of perforation of a blood vessel by the distal end tip 50.

Furthermore, as illustrated in FIG. 2(a), the outer peripheral surface of the distal end tip 50 of the first embodiment is covered with outer coating 60a. Here, in the catheter 1 of the first embodiment, the outer coating 60a enters the inside of the slit 52 of the distal end tip 50.

In such a catheter 1 of the first embodiment, the metallic distal end tip 50 is provided with the slit 52, thus allowing the distal end tip 50 to bend easily while the hardness thereof is maintained. Moreover, the outer peripheral surface of the distal end tip 50 is covered with the outer coating 60a, which smooths the surface of the distal end tip 50. As a result, it is possible to realize the catheter 1 being capable of passing a hard lesion easily, having favorable following properties for a tortuous blood vessel such as a peripheral blood vessel, and making it difficult for the distal end tip 50 to be caught on a lesion, for example.

Particularly, in the catheter 1 of the first embodiment, the outer coating 60a covering the distal end tip 50 enters the inside of the slit 52 to be firmly fixed to the distal end tip 50. Therefore, it is possible to prevent separation of the outer coating 60a from the distal end tip 50 due to friction with the outside. Consequently, even when the distal end tip 50 is made to pass a calcified lesion, for example, the smooth surface of the distal end tip 50 can be securely maintained.

### B. Second embodiment

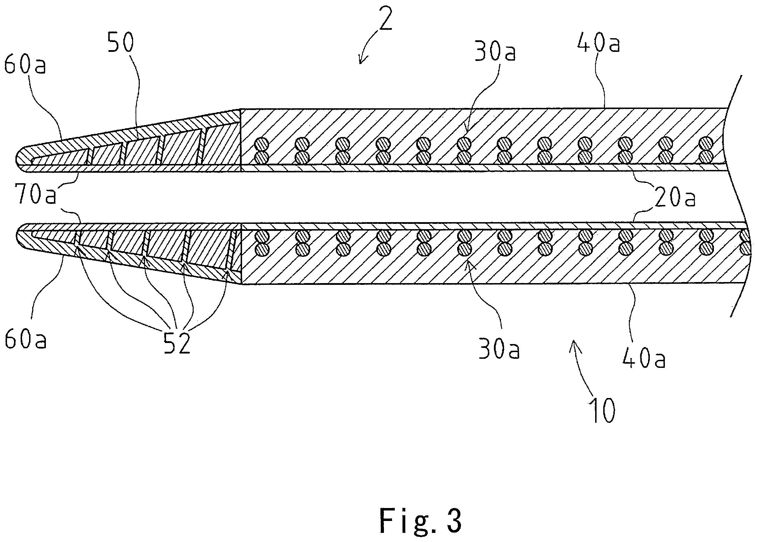

FIG. 3 is an enlarged view illustrating the vicinity of a distal end of a catheter 2 according to a second embodiment of the invention. The illustrated catheter 2 of the second embodiment is different from the catheter 1 of the above-described first embodiment in the following aspect. That is, the outer coating 60a is provided on the outer peripheral surface of the distal end tip 50 and, in addition, inner coating 70a is provided on the inner surface of the distal end tip 50. Then, the outer coating 60a entering the inside of the slit 52 of the distal end tip 50 reaches the inner coating 70a, so that the outer coating 60a and the inner coating 70a are in close contact with each other.

Regarding aspects other than the above, the catheter 1 of the first embodiment and the catheter 2 of the second embodiment have the same structure. That is, the catheter shaft 10 is constituted by the inner layer 20a, the braid layer 30a covering the inner layer 20a, and the outer layer 40a covering the braid layer 30a.

Also in such a catheter 2 of the second embodiment, it is possible to maintain easiness in bending of the metallic distal end tip 50 and prevent the case in which the distal end tip 50 is caught on a lesion, for example, similarly to the catheter 1 of the above-mentioned first embodiment.

Moreover, in the catheter 2 of the second embodiment, the distal end tip 50 is covered with the outer coating 60a and the inner coating 70a, and the outer coating 60a entering the inside of the slit 52 and the inner coating 70a come in contact with each other. Thus, the outer coating 60a (and the inner coating 70a) can be fixed to the distal end tip 50 more firmly.

Furthermore, the inner coating 70a can also smoothen the inner surface of the distal end tip 50, which also prevents the case in which a medical instrument (a guide wire, for example) passing a lumen of the catheter 2 is caught on the inner peripheral surface of the distal end tip 50.

### C. Third embodiment:

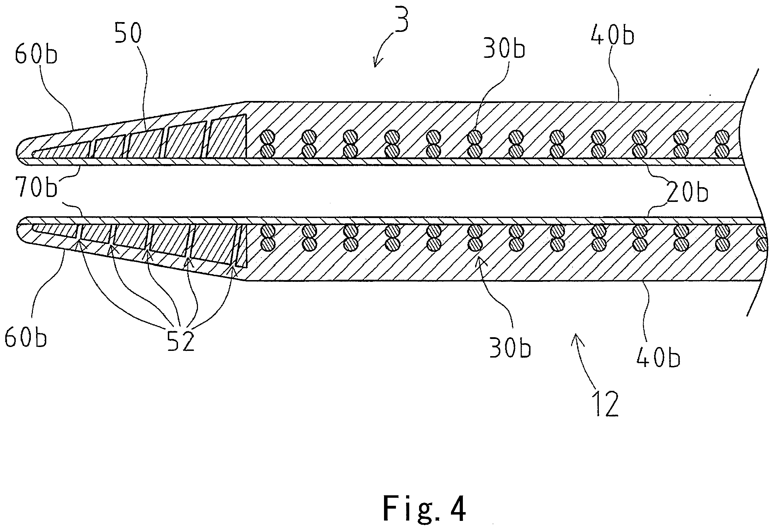

FIG. 4 is an enlarged view illustrating the vicinity of a distal end of a catheter 3 according to a third embodiment of the invention. The catheter 3 of the third embodiment is different from the catheter 2 of the above-described second embodiment in the following aspect. That is, in the catheter 3 of the third embodiment, an outer layer 40b of a catheter shaft 12 and outer coating 60b covering the outer peripheral surface of the distal end tip 50 are formed integrally, and an inner layer 20b of the catheter shaft 12 and inner coating 70b covering the inner surface of the distal end tip 50 are formed integrally.

Regarding aspects other than the above, the catheter 3 of the third embodiment has the same structure as the catheter 2 of the above-described second embodiment. That is, the catheter shaft 12 is provided with a braid layer 30b covering the inner layer 20b, and the distal end tip 50 is provided with the slit 52.

Also in such a catheter 3 of the third embodiment, it is possible to maintain easiness in bending of the metallic distal end tip 50 and prevent the case in which the distal end tip 50 is caught on a lesion, for example, similarly to the catheters of the various embodiments described above.

Moreover, in the catheter 3 of the third embodiment, the outer coating 60b and the outer layer 40b are formed integrally, and the inner coating 70b and the inner layer 20b are formed integrally, whereby the connection portion between the distal end tip 50 and the catheter shaft 12 is coated seamlessly. This prevents occurrence of catch with the outside (a lesion, for example) and the inside (a guide wire passing a lumen of the catheter, for example) at the connection portion between the distal end tip 50 and the catheter shaft 12. At the same time, it is also possible to enhance connection strength between the distal end tip 50 and the catheter shaft 12.

### D. Fourth embodiment:

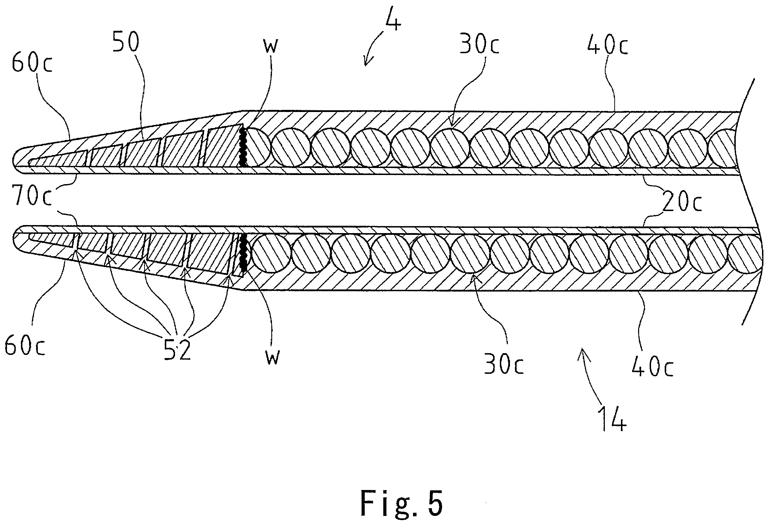

FIG. 5 is an enlarged view illustrating the vicinity of a distal end of a catheter 4 according to a fourth embodiment of the invention. The catheter 4 of the fourth embodiment is different from the catheter 3 of the above-described third embodiment in the following aspect. That is, the catheter 4 of the fourth embodiment includes, as a reinforcing body, a coil body 30c formed by winding metallic wires into a helical coil structure. Then, the metallic distal end tip 50 is welded to the distal end portion of the coil body 30c. In FIG. 5, a welded portion w between the coil body 30c and the distal end tip 50 is illustrated by a bold wave line.

Note that the coil body 30c may be formed by winding a single wire into a helical coil structure (a so-called single wound coil) or may be a hollow body formed by winding a plurality of wires into a helical coil structure (a so-called stranded wire coil). Considering the rotation force of the catheter 4, it is preferable to use a stranded wire coil as the coil body 30c. Moreover, the metallic material of the wires of the coil body 30c is not especially limited, and there can be used stainless steel (SUS304, SUS316, etc.), gold, white gold, tungsten, platinum, nickel, and an alloy of such elements, for example.

Regarding aspects other than the above, the catheter 4 of the fourth embodiment has the same structure as the catheter 3 of the above-described third embodiment. That is, a catheter shaft 14 has an inner layer 20c and an outer layer 40c. Moreover, the distal end tip 50 is provided with the slit 52. The outer peripheral surface of the distal end tip 50 is covered with outer coating 60c, and the inner surface thereof is covered with inner coating 70c.

Also in such a catheter 4 of the fourth embodiment, it is possible to maintain easiness in bending of the metallic distal end tip 50 and prevent the case in which the distal end tip 50 is caught on a lesion, for example, similarly to the catheters of the various embodiments described above.

Moreover, in the catheter 4 of the fourth embodiment, the reinforcing body is formed as the metallic coil body 30c, and the coil body 30c and the distal end tip 50 are welded to each other. Thus, it is possible to further enhance connection strength between the distal end tip 50 and the catheter shaft 14.

### E: Fifth embodiment:

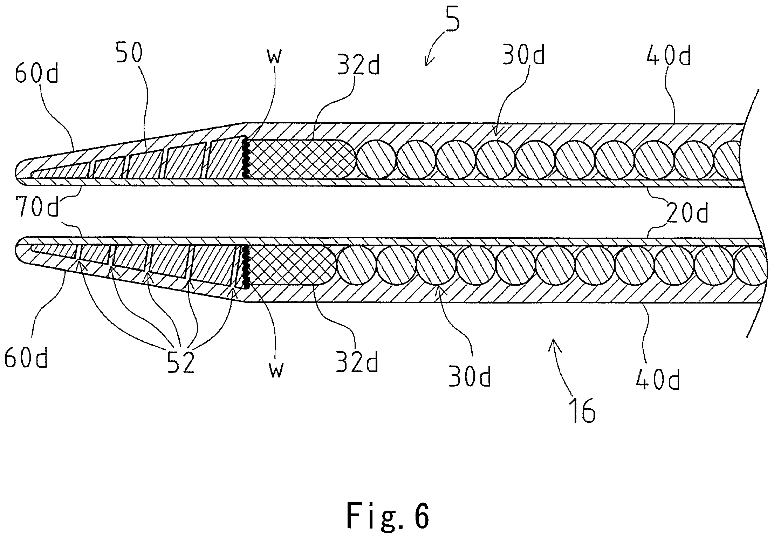

FIG. 6 is an enlarged view illustrating the vicinity of a distal end of a catheter 5 according to a fifth embodiment of the invention. The catheter 5 of the fifth embodiment is different from the catheter 4 of the above-described fourth embodiment in the following aspect. That is, in the catheter 5 of the fifth embodiment, the distal end of a coil body 30d includes a part formed integrally by mutually-melted wires constituting the coil body 30d (a melted portion 32d), and the distal end tip 50 is welded to the melted portion 32d.

Regarding aspects other than the above, the catheter 5 of the fifth embodiment has the same structure as the catheters of the embodiments described above. That is, a catheter shaft 16 has an inner layer 20d and an outer layer 40d. Moreover, the distal end tip 50 is provided with the slit 52. The outer peripheral surface of the distal end tip 50 is covered with outer coating 60d and the inner surface thereof is covered with inner coating 70d.

Also in such a catheter 5 of the fifth embodiment, it is possible to maintain easiness in bending of the metallic distal end tip 50 and prevent the case in which the distal end tip 50 is caught on a lesion, for example, similarly to the catheters of the various embodiments described above.

Moreover, in the catheter 5 of the fifth embodiment, the wires are melted mutually and formed integrally at the distal end portion of the coil body 30d, which regulates the movement of the wires at the distal end portion of the coil body 30d. For example, when a stranded wire coil (a hollow body formed by winding a plurality of wires into a helical coil structure) is used as the coil body 30d, it is possible to prevent loosening of the end portion of the stranded wire coil. As a result, it is possible to easily join the distal end tip 50 and the distal end portion of the coil body 30d.

## Claims

1. A catheter (1; 2; 3; 4; 5) comprising:
a tube body (10) including an inner layer (20a), a reinforcing body (30a; 30b; 30c; 30d) covering the inner layer (20a), and an outer layer (40a) covering the reinforcing body (30a; 30b; 30c; 30d); and
a tubular metallic distal end tip (50) provided at a distal end of the tube body (10), **characterized in that**
a slit (52) is formed on the distal end tip (50) and penetrates the distal end tip (50) from an outer peripheral surface of the distal end tip (50) to an inner surface of the distal end tip (50), and
an outer peripheral surface of the distal end tip (50) is provided with an outer coating (60a), and the outer coating (60a) enters inside of the slit (52).

2. The catheter (2; 3) according to claim 1, wherein
an inner peripheral surface of the distal end tip (50) is provided with an inner coating (70a), and
the outer coating (60a) entering the inside of the slit (52) and the inner coating (70a) come in contact with each other.

3. The catheter (3) according to claim 2, wherein
the outer coating (60a) and the outer layer (40a) of the tube body (10) are formed integrally, and the inner coating (70a) and the inner layer (20a) of the tube body (10) are formed integrally.

4. The catheter (1; 2; 3) according to any one of claims 1 to 3, wherein
the reinforcing body (30a; 30b) is a braid layer.

5. The catheter (4; 5) according to any one of claims 1 to 3, wherein
the reinforcing body (30c; 30d) is a coil body formed by winding at least one metallic wire into a helical coil structure, and
the distal end tip (50) is welded to a distal end of the coil body (30c; 30d).

6. The catheter (4, 5) according to claim 5, wherein
the coil body (30c; 30d) is formed by winding a single metallic wire.

7. The catheter (4, 5) according to claim 5, wherein
the coil body (30c; 30d) is formed by winding a plurality of metallic wires.

8. The catheter (5) according to claim 7, wherein
the distal end of the coil body (30d) includes a part (32d) formed integrally by mutually-melted wires constituting the coil body (30d), and the metallic distal end tip (50) is welded to the part (32d).

9. The catheter (1; 2; 3; 4; 5) according to any one of claims 1 to 8, wherein
the slit (52) formed on the distal end tip (50) is formed helically.

10. The catheter (1; 2; 3; 4; 5) according to claim 9, wherein
a pitch of the slit (52) decreases toward a distal end of the distal end tip (50).

## Patentansprüche

1. Katheter (1; 2; 3; 4; 5) mit:
einem Rohrkörper (10), der eine Innenschicht (20a), einen die Innenschicht (20a) ummantelnden Verstärkungskörper (30a; 30b; 30c; 30d) und eine den Verstärkungskörper (30a; 30b; 30c; 30d) ummantelnde Außenschicht (40a) aufweist; und
einer rohrförmigen metallischen distalen Spitze (50), die an einem distalen Ende des Rohrkörpers (10) vorgesehen ist, **dadurch gekennzeichnet, dass**
in der distalen Spitze (50) ein Schlitz (52) ausgebildet ist, der die distale Spitze (50) von ihrer Außenumfangsfläche bis zu ihrer Innenumfangsfläche durchdringt, und
die Außenumfangsfläche der distalen Spitze (50) mit einer in den Schlitz (52) eindringenden Außenbeschichtung (60a) versehen ist.

2. Katheter (2; 3) nach Anspruch 1, wobei
die Innenumfangsfläche der distalen Spitze (50) mit einer Innenbeschichtung (70a) versehen ist, und
die in den Schlitz (52) eindringende Außenbeschichtung (60a) und die Innenbeschichtung (70a) miteinander in Kontakt sind.

3. Katheter (3) nach Anspruch 2, wobei
die Außenbeschichtung (60a) und die Außenschicht (40a) des Rohrkörpers (10) integral ausgebildet sind, und
die Innenbeschichtung (70a) und die Innenschicht (20a) des Rohrkörper (10) integral ausgebildet sind.

4. Katheter (1; 2; 3) nach einem der Ansprüche 1 bis 3, wobei
der Verstärkungskörper (30a; 30b) eine Geflechtschicht ist.

5. Katheter (4; 5) nach einem der Ansprüche 1 bis 3, wobei
der Verstärkungskörper (30c; 30d) ein Wicklungskörper ist, der aus wenigstens einem zu einer schraubenförmigen Wicklungsstruktur gewickelten Metalldraht gebildet ist, und
die distale Spitze (50) mit dem distalen Ende des Wicklungskörpers (30c; 30d) verschweißt ist.

6. Katheter (4, 5) nach Anspruch 5, wobei
der Wicklungskörper (30c; 30d) aus einem einzelnen gewickelten Metalldraht gebildet ist.

7. Katheter (4, 5) nach Anspruch 5, wobei
der Wicklungskörper (30c; 30d) aus einer Vielzahl gewickelter Metalldrähte gebildet ist.

8. Katheter (5) nach Anspruch 7, wobei
das distale Ende des Wicklungskörpers (30d) einen Teil (32d) aufweist, der aus miteinander verschmolzenen Drähten, aus denen der Wicklungskörper (30d) gebildet ist, integral ausgebildet ist, und
die metallische distale Spitze (50) mit dem Teil (32d) verschweißt ist.

9. Katheter (1; 2; 3; 4; 5) nach einem der Ansprüche 1 bis 8, wobei
der in der distalen Spitze (50) ausgebildete Schlitz (52) schraubenförmig ausgebildet ist.

10. Katheter (1; 2; 3; 4; 5) nach Anspruch 9, wobei
die Ganghöhe des Schlitzes (52) zum distalen Ende der distalen Spitze (50) hin abnimmt.

## Revendications

1. Cathéter (1 ; 2 ; 3 ; 4 ; 5) comprenant :
un corps tubulaire (10) comprenant une couche interne (20a), un corps de renfort (30a ; 30b ; 30c ; 30d) recouvrant la couche interne (20a), et une couche externe (40a) recouvrant le corps de renfort (30a ; 30b ; 30c ; 30d) ; et
un embout d'extrémité distale métallique tubulaire (50) disposé à une extrémité distale du corps tubulaire (10), **caractérisé en ce que** :
une fente (52) est formée sur l'embout d'extrémité distale (50) et pénètre dans l'embout d'extrémité distale (50) à partir d'une surface périphérique externe de l'embout d'extrémité distale (50) jusqu'à une surface interne de l'embout d'extrémité distale (50), et
une surface périphérique externe de l'embout d'extrémité distale (50) est pourvue d'un revêtement externe (60a), et le revêtement externe (60a) pénètre à l'intérieur de la fente (52).

2. Cathéter (2 ; 3) selon la revendication 1, dans lequel
une surface périphérique interne de l'embout d'extrémité distale (50) est pourvue d'un revêtement interne (70a), et
le revêtement externe (60a) pénétrant à l'intérieur de la fente (52) et le revêtement interne (70a) viennent en contact l'un avec l'autre.

3. Cathéter (3) selon la revendication 2, dans lequel
le revêtement externe (60a) et la couche externe (40a) du corps tubulaire (10) sont formés d'un seul tenant, et le revêtement interne (70a) et la couche interne (20a) du corps tubulaire (10) sont formés d'un seul tenant.

4. Cathéter (1 ; 2 ; 3) selon l'une quelconque des revendications 1 à 3 dans lequel
le corps de renfort (30a ; 30b) est une couche tressée.

5. Cathéter (4 ; 5) selon l'une quelconque des revendications 1 à 3, dans lequel
le corps de renfort (30c ; 30d) est un corps de bobine formé en enroulant au moins un fil métallique en une structure de bobine hélicoïdale, et
l'embout d'extrémité distale (50) est soudé à une extrémité distale du corps de bobine (30c ; 30d).

6. Cathéter (4, 5) selon la revendication 5, dans lequel
le corps de bobine (30c ; 30d) est formé en enroulant un fil métallique unique.

7. Cathéter (4,5) selon la revendication 5, dans lequel
le corps de bobine (30c, 30d) est formé en enroulant une pluralité de fils métalliques.

8. Cathéter (5) selon la revendication 7, dans lequel
l'extrémité distale du corps de bobine (30d) comprend une partie (32d) formée d'un seul tenant par des fils mutuellement fondus constituant le corps de bobine (30d), et l'embout d'extrémité distale métallique (50) est soudé à la partie (32d).

9. Cathéter (1 ; 2; 3; 4; 5) selon l'une quelconque des revendications 1 à 8, dans lequel
la fente (52) formée sur l'embout d'extrémité distale (50) est conformée en hélice.

10. Cathéter (1 ; 2 ; 3 ; 4 ; 5) selon la revendication 9, dans lequel
un pas de la fente (52) diminue vers une extrémité distale de l'embout d'extrémité distale (50).
